# EUROPEAN PATENT APPLICATION

(11) **EP 4 686 460 A1**
(43) Date of publication of application: **04.02.2026**
(21) Application number: 24191849.9
(22) Date of filing: 30.07.2024
(51) Int. Cl.: A61F 9/007, A61F 2/08, A61L 27/50, H10N 30/20

(54) **ELECTROMECHANICAL DEVICE FOR ACTUATING A PATIENT'S EYELID**

(71) Applicant: Ecole Polytechnique Fédérale de Lausanne (EPFL), 1015 Lausanne (CH)
(72) Inventor: Konstantinidi, Stefania Maria Aliki, 1166 Perroy (CH); Civet, Yoan, 74500 Publier (FR); Perriard, Yves, 2000 Neuchâtel (CH)
(74) Representative: P&TS SA (AG, Ltd.)

(57) **Abstract**

Electromechanical implantable device for actuating a patient's eyelid (11), comprising an actuator (2) for activating the opening or closing of the eyelid.

The actuator is a dielectric elastomer actuator.

The device may comprise a rod (3). The actuator is intended to be implanted under the skin of the patient's forehead, and linked to the eyelid via said rod.

## Description

### Technical domain

The present invention concerns an electromechanical device for actuating a patient's eyelid. Such a device can be used for the restoration eye blinking for patients affected by a facial paralysis.

### Related art

Facial paralysis, a condition characterized by the loss of voluntary muscle movement in the face, can arise from several etiologies, including but not limited to nerve damage due to congenital conditions, neurological disorders such as multiple sclerosis, stroke, trauma, tumors exerting a pressure on the facial nerve, or diseases such as Bell's Palsy or the Lyme disease, among others.

One of the critical issues associated with facial paralysis is the inability to blink. Blinking, an essential physiological function, protects and maintains the health of the eye by spreading tears, removing irritants, and preventing dryness.

The inability to blink, medically termed lagophthalmos, has significant repercussions on ocular health if left unaddressed:
- Dry eye syndrome: Blinking distributes the tear film across the surface of the eye, essential for lubrication. Impaired blinking results in insufficient tear distribution, causing dryness and discomfort.
- Exposure keratitis: Without the protective mechanism of blinking, the cornea remains exposed to environmental irritants, leading to corneal epithelial damage or corneal ulceration.
- Infection risk: The absence of regular blinking increases the likelihood of infections due to the accumulation of debris and pathogens on the eye surface.
- Visual impairment: Chronic corneal damage and ulceration can lead to permanent visual impairment or blindness if not adequately managed.

Common solutions to mitigate those risks include using goggles or an occlusion bandage on the affected eye; tarsorrhapy (a surgical procedure that partially sews the eyelids together to reduce the size of opening between the eyelids); or Cross-Facial Nerve Grafting (CFNG). While those methods may reduce the risk of secondary complications, they have a significant impact on the patients' quality of life.

Addressing the inability to blink in patients with facial paralysis is therefore crucial to overcome those drawbacks. Research and development of effective solutions to restore or simulate the blinking function are imperative to improve the outcomes for patients suffering from lagophthalmos.

DE19632392A1 discloses an active eye prosthesis with motorised upper lid movement. It requires replacement of the complete eyeball with a glass eye, and is not adapted for patient having a functional eye that does not need replacement.

BR102015006668A2 discloses a motorized eyelid prosthesis actuated by an electric motor, such as a rotary or linear motion DC motor. Such a conventional, coil based motor generates heat that is difficult to evacuate, and requires a volume that is hardly available anywhere near the eyelid.

JPS6041976Y2 discloses a device that can be used to forcibly open the eyelids to expose the eyeballs to the outside, and then place a predetermined pattern directly facing the eyeballs. This device requires a cumbersome motor attached to the head of the user; it is not adapted s a permanent implant.

It has also been suggested to develop artificial muscles to restore the blinking function of patients. Artificial muscles function through actuators that produce motion of contraction and expansion, similar to the ones of natural biological muscles. Several artificial muscle technologies are currently under research and application in robotics and prosthetics, including Pneumatic Artificial Muscles (PAMs) and Shape Memory Alloys (SMAs). PAMs and SMAs, however, face limitations due to their operational principles, resulting in low actuation frequencies. Moreover, PAMs are cumbersome and require pumps and compressors, while SMAs present lower compliance and slow response times.

### Short disclosure of the invention

An aim of the present invention is the provision of another implantable device for restoring the blinking function.

According to the invention, these aims are attained by the object of the attached claims, and especially with an electromechanical implantable device for actuating a patient's eyelid, comprising an actuator for activating the opening or closing of the eyelid,
characterised in that said actuator is a dielectric elastomer actuator.

Dielectric Elastomer Actuators (DEAs) is a recent type of EAP that converts electrical energy into mechanical work. DEAs excel as soft actuators due to their ability to actuate in multiple directions, exhibit large strains, and possess high energy density. The use of a dielectric elastomer actuator thus makes it possible to build an actuator that is not very bulky, and in any case not very thick, which can therefore be placed easily under the patient's skin and be almost invisible.

Such an actuator generates little heat that can easily be evacuated.

The device may comprise a rod. The actuator is preferably intended to be implanted under the skin of the patient's forehead, and linked to the patient's eyelid via said rod.

The shape of the rod is irrelevant as long as it can connect the actuator with the eyelid and be rigid enough to close or open the eyelid.

The actuator is preferably flexible so that it can be put against the patient's skull and adapted to the shape of this skull.

The DEA may consist of a silicone layer sandwiched between two conductive electrodes: when a voltage is applied on the electrodes, a compressive Maxwell stress is generated, inducing a thickness reduction and thus an expansion in the other dimensions.

The device preferably comprises a spring for controlling the displacement of the actuator as a function of the force exerted by said actuator.

The spring may have a negative stiffness over at least one part of its operating range.

The derivative of the displacement-force function of the spring may be positive over a portion of an actuator trajectory when said actuator is actuated, and negative over another portion.

The spring may be housed in or under the eyelid. It is thus invisible, or almost invisible, when implanted.

The spring could be in the shape of a beam arranged to be placed in the ciliary bundle on the extremity of the eyelid.

The DEA could be attached through the rod to the center of the beam and placed on the forehead.

The spring may have a curved shape with one inflexion point at one extremity of the displacement of said actuator, and a curve with three inflexion points between the two extremities of said displacement.

Each extremity of the spring may be fixed to the patient's skull at each side of the eye.

The dielectric elastomer actuator may comprise a flexible sheet whose length and width vary according to the voltage applied.

For the restoration of blinking abilities, a unidirectional actuation of the actuator is needed.

The deformation of this sheet is preferably anisotropic in the plane of the actuator. It is preferably larger in the main direction of closure of the eyelid than in an orthogonal direction.

The dielectric elastomer actuator may be reinforced with fibres. The fibres are oriented so as to control the anisotropy of its deformation.

The movement in the direction of the fibers is constrained, thus enhancing the actuation in the perpendicular direction, i.e., in the direction of actuation of the eyelid.

The fibres are preferably 3D printed.

The fibres may extend straight or curved in a substantially horizontal direction when the patient is standing.

The device is preferably arranged so that the eyelid remains open when no voltage is applied to said dielectric.

The device may have two stable positions, one corresponding to the closed eyelid and the other one to an open eyelid.

The eyelid preferably remain open or closed when no voltage is applied to said dielectric, and switch from one state to the other with a suitable control voltage. The device thus uses no energy most of the time, when the patient's eye are open.

The device may be arranged so that said the eyelid moves in a direction when the patient is standing comprising a main vertical component as well as a horizontal component.

The device may comprise a battery intended to be worn on the patient chest or behind the patient neck.

The actuator, the rod and the spring are adapted to be implanted in the patient's body.

The device may comprise a neural interface controlled by the nerve controlling said patient's other eyelid.

The actuator on the paralyzed side of the face could be synchronized with the activity of the healthy contralateral side. Signals from the healthy side a drawn to the paralyzed side to achieve symmetric movements. By enabling real-time control of the actuator from the healthy hemiface, this prosthesis would allow patients to symmetrically blink. A significant advantage of this method over traditional therapies is that it eliminates the need for nerve grafts or muscle flaps, thereby avoiding the lengthy waiting periods between surgical procedures and eliminating the need for surgical intervention on other parts of the body.

### Short description of the drawings

Exemplar embodiments of the invention are disclosed in the description and illustrated by the drawings in which:
Figure 1 illustrates a perspective view of a human's face with a device according to one embodiment of the invention;
Figures 2A and 2B schematically illustrate a human face with a device according to the invention, with the eyelid open respectively closed.
Figures 3A and 3B illustrates the deformation of a DEA when a voltage is applied;
Figure 4 illustrates an actuator that could be used in the device of the invention;
Figure 5A to 5G illustrate the deformations of the spring when a force Fc is applied;
Figure 6 is a chart that illustrates the force as a function of the displacement of the eyelid.

### Examples of embodiments of the present invention

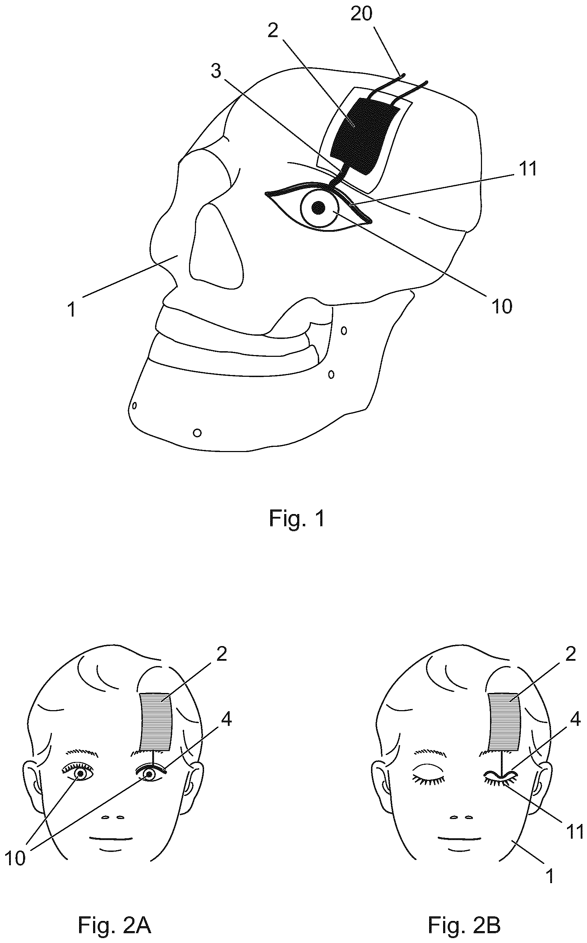

Figures 1 and 2A - 2B illustrate a perspective view of a human face 1 equipped with an electromechanical implanted device for actuating a patient's eyelid 11 according to one embodiment of the invention. The device comprises an actuator 2 for moving the eyelid 11 to open or close the eye 10. The actuator 2 exerts an opening or closing force on the eyelid 11 through a rod 3 attached on one side to the actuator 2 and on the other side to a spring 4 under the eyelid.

The actuator 2 deforms when voltage is applied via electrical wires 20. The energy is supplied by an electrical source, for example a battery, which can be implanted in the patient's body, or worn against the skin, for example on the patient chest or behind the patient neck.

A neural interface may be provided for controlling the actuator from the nerve controlling the patient's other eyelid.

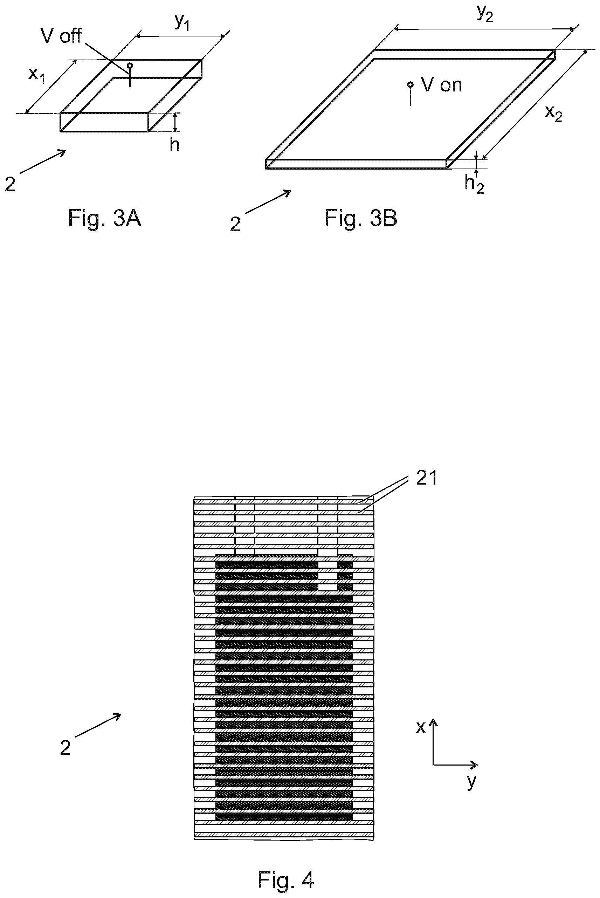

Figures 3A and 3B illustrate the deformations of the actuator 2 when a voltage V is applied. In the absence of voltage (Voff), i.e. in the situation illustrated in Figure 3A, the actuator has a width y1, a length x1, and a thickness h1. When a voltage Von is applied (Figure 3B), actuator 2 deforms in all dimensions and its dimensions become y2, x2 and h2 respectively.

The actuator 2 may be produced by stacking modules, where each module may comprise one silicone layer or other known electroactive polymers as active layer. At least one silver contact of 2 mm width is screen printed to have a good charge distribution and the lowest electrical resistance. A carbon based electrode is then screen printed on top of the active layer and contact. The electrode may comprise a mixture of carbon, isopropanol and silicone.

A module may comprise one or a plurality of layers of silicone with variable thickness. Inner active layers, between a negative and a positive electrode, may be between 30 and 300 *µ*m thick, preferably between 50 and 150 *µ*m, for example 100 *µ*m. External encapsulation layers may be thinner in order to avoid stiffening the actuators. They may be between 5 and 50 *µ*m, preferably between 10 and 30 *µ*m, for example 20 *µ*m.

Multiple such modules may be stacked on top of each other, for example using silicone glue doped with carbon black particles. A positive electrode is preferably located between the two negative electrodes, which may be grounded, in order to confine the electrical field. The contacts of the actuators are established by connecting copper wires to the silver tracks of each module.

In one embodiment, the actuator comprises two active layers which are sandwiched by three electrodes.

A base silicone layer may be used and act as encapsulation on one side of the device. The active layers may be stacked on top of a base layer. The thickness of the base layer may be lower than the thickness of the other active layer.

The upper surface of the actuator may also be covered with an encapsulation layer, such as a silicone layer.

Other materials of surgical grade could be used for the encapsulation.

The actuator 2 may have a square, rectangular or any other surface. In one preferred embodiment, the actuator 2 has a substantially rectangular surface, with a dimension along the actuation axis X larger than the dimension along the perpendicular axis Y. The dimension of the actuator is preferably between 10x10 and 100x100mm, for example 20x50 mm. Its total thickness is preferably between 100 and 500 *µ*m, for example 300 *µ*m.

The contacts of the actuators are established by connecting copper wires 20 to the silver electrodes of each module. A voltage can be applied between the electrodes to modify the viscoelastic properties and/or the dimensions of the polymer layer. As shown on Figure 3, in an example, the applied voltage induces a contraction in the thickness direction and an expansion in-plane. The closure of the eyelid is preferably obtained by applying a voltage so as to expand the actuator in the closure direction; therefore, when no voltage is applied, the eyelid is open. The stability of the open position is reinforced by the spring, as will be described. Therefore, when most of the time the eye is open, the device does not require any power.

For the restoration of blinking abilities, a unidirectional actuation of the actuator along the X axis is sufficient. Therefore, the deformation of the actuator 2 is preferably anisotropic. It is preferably larger in the main direction of closure of the eyelid than in an orthogonal direction.

As shown in Figure 4, actuator 2 advantageously comprises fibers 20 along the y axis perpendicular to the direction of actuation, in order to limit the deformations along this axis. By restricting deformations along the Y axis, deformations along the X axis are increased.

To create the anisotropy, passive stiff fibers 21 are placed within or on top of the actuator 2, as shown on Figure 4.

Fibers may also be placed on top of each module, if the actuator comprises multiple modules.

The fibers constrain the deformation in one direction, enhancing the resulting strain in the perpendicular direction.

The fibers 21 may be made out of PET, or other suitable polymer. The fibers 21 may be laser-cut out of ploymer sheets.

Silicone glue is applied onto the previously fabricated actuators. The fibers are then deposited onto the uncured silicone glue, and the final actuator is cured, for example during 2 hours at 80°C. The silicone acts as an encapsulation layer on top of the actuator.

To increase the displacement that can be achieved with a given actuator and a given voltage, the actuator 2 is preferably paired with a biasing system, preferably a mechanical biasing system, such as a constant load or a spring.

As shown on Figures 1 and 2, the biasing system preferably comprises a mechanical spring 4 mounted under or within the eyelid, and attached to the actuator 2 through a rod 3 that transmits the displacement of the actuator 2 to the spring 4 and to the eyelid.

The spring 4 is preferably a non-linear spring, i.e. a spring whose deformation is not linearly proportional to the force applied by the actuator in the useful range. By using a nonlinear spring, the stroke is significantly enhanced compared to a conventional load.

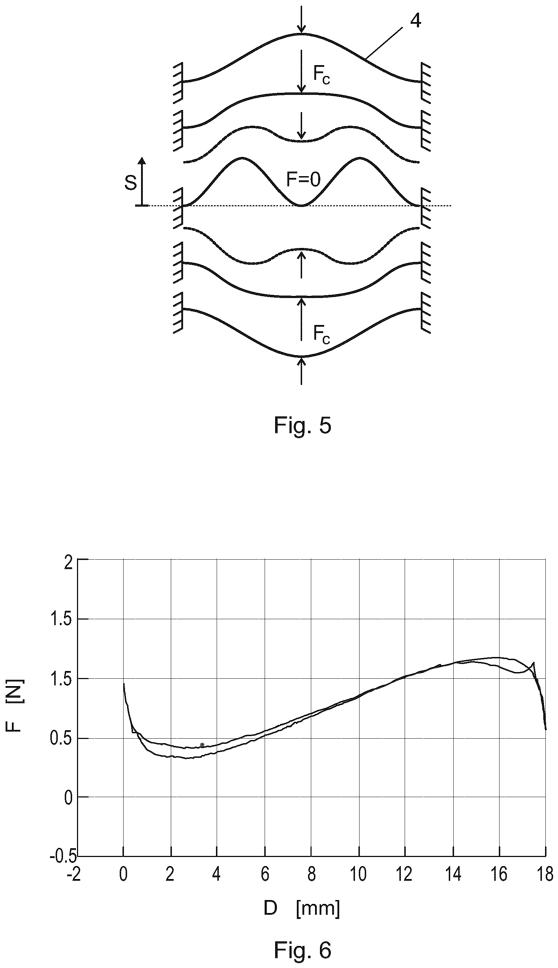

The spring 4 has preferably a negative stiffness over at least one part of its operating range. In other words, as shown on Figure 6, the displacement D on the portion of the spring 4 to which the rod 3 is attached is inversely proportional to the force F exerted by the actuator 2, at least for a portion of the stroke when the spring has left its stable state. Over that range, the force exerted by the spring 3 helps for the displacement

The spring 4 may have one single stable position, two stable positions, or three stable positions. A spring with a single stable position is a spring which is shaped and/or mounted so that it returns to a single, normal, stable position when no additional external forces are applied. A spring with two or three stable positions is a spring which is shaped and/or mounted so that it returns to either one of those stable positions, usually the closest position, when no additional external forces are applied

Figure 5 illustrates an example of spring 4 that could be used within the invention.

The spring 4 is mounted under the eyelid so that movements of the midpoint of the eyelid are transmitted to the eyelid. The spring could be in the shape of a beam arranged to be placed in the ciliary bundle on the extremity of the eyelid. The two ends 40 and 41 of the spring are fixed and, for example, attached to the patient's skull on either side of the eyelid 11. The spring 4 forms a beam pre-compressed between its two ends.

This spring may have three stable positions.

In the position shown in Figure 5A, corresponding, for example, to an open eyelid, spring 4 bulges upwards, with a single point of inflection where the rod is attached. A force Fc exerted on the middle of the spring by the actuator 2 via the rod 3 deforms the spring 4 through the intermediate positions of Figures 5B and 5C, until it reaches the barely stable intermediate position of Figure 5D. In the position shown in Figure 5D, the spring 4 forms a curve with two vertices and a low point between these two vertices. The eyelid may be half-closed in this position. In another embodiment, the eyelid is closed in that position.

By continuing to exert a downward force, spring 4 passes through the unstable intermediate positions of Figures 5E and 5F, before reaching the stable position of Figure 5G, corresponding to closure of the eyelid. In the position shown in Figure 5G, the spring 4 is bulged downwards, with a single point of inflection where the rod is attached.

The opening of the eyelid, passing from the state of Figure 5G to that of Figure 5A, is obtained by exerting a force Fc opposite to that required for closure.

The final displacement reached is preferably between 2 and 6mm, in the range of the pupil's dimensions.

The displacement of the eyelid is mainly vertical when the patient is standing. In one embodiment, this displacement comprises an horizontal component, so that the eyelid sweeps the eye when it opens or closes.

In one embodiment, the travel of spring 4 is restricted and the spring can only move between the positions shown in Figures 5B and 5F, for example, or between 5B and 5D, being mechanically prevented from reaching the extreme positions above and below. This prevents the spring from snapping.

The eyelid can be held open without exerting any force on the spring 4, i.e. with little or no tension on the actuator 2. Voltage between the actuator electrodes needs to be applied to move from the open state in Figure 5A to the closed state in Figure 5G, or vice versa.

If the closed position of the eyelid corresponds to a stable state, for example the stable state of Figure 5G, the eyelid can be held closed without exerting any force on the spring 4, i.e. with little or no tension on the actuator 2. If, in another embodiment, the closed position of the eyelid corresponds to an instable state, a voltage needs to be applied to maintain the eyelid closed.

The spring 4 may be fabricated by laser cutting a metal sheet to the desired shape. The dimensions of the spring is determined by considering the diameter of the orbicularis oculi muscle and the space available inside the eyelid. The length of the spring for example between 50 and 80 mm as it should be longer than the orbicularis oculi to guide the eyelid in the open and closed positions.

The eyelid 11 may be the patient eyelid. Since it may be difficult to mount the spring 4 under or within an existing eyelid, the eyelid 11 may be an artificial eyelid.

## Claims

1. Electromechanical implantable device for actuating a patient's eyelid (11), comprising an actuator (2) for activating the opening or closing of the eyelid,
**characterised in that** said actuator is a dielectric elastomer actuator.

2. Device according to claim 1, comprising a rod (3), said actuator being intended to be implanted under the skin of the patient's forehead, and linked to the eyelid via said rod.

3. Device according to one of claims 1 to 2, said actuator (2) being flexible so that it can be pressed against the patient's skull (1).

4. Device according to one of claims 1 to 3, comprising a spring (4) for controlling the displacement of said eyelid (11) as a function of the force exerted by said actuator.

5. Device according to claim 4, said spring (4) having a negative stiffness over at least one part of its operating range.

6. Device according to claim 4, said spring (4) having at least one stable state where it forms one curve with one single inflexion point, and another state where it forms a curve with three inflexion points.

7. Device according to one of the claims 1 to 6, arranged so that the eyelid (11) remains open when no voltage is applied to the dielectric elastomer actuator.

8. Device according to claim 7, arranged so that the eyelid (11) closes when a voltage is applied to the dielectric elastomer actuator.

9. Device according to one of claims 4 to 8, said spring (4) being accommodated in or under the eyelid (11).

10. Device according to claim 9, each extremity (40, 41) of said spring (4) being intended to be fixed to the patient's skull at each side of the eye.

11. Device according to one of claims 1 to 10, said dielectric elastomer actuator (2) comprising a flexible sheet whose length and width vary according to the voltage applied, the deformation of said sheet (2) being anisotropic in the plane of the sheet.

12. Device according to claim 11, said dielectric elastomer actuator (2) comprising fibres (21) oriented so as to control the anisotropy of its deformation.

13. Device according to claim 12, said fibres (21) being 3D printed.

14. Device according to one of the claims 1 to 13, said fibres (21) extending straight or curved in a substantially horizontal direction when the patient is standing.

15. Device according to one of the claims 1 to 14, arranged so that said eyelid moves in a direction comprising an horizontal component when the patient is standing.

16. Device according to one of the claims 1 to 15, comprising a neural interface arranged for being controlled by the nerve controlling said patient's other eyelid.
